(19) Europäisches Patentamt
European Patent Office
Office européen des brevets

(11) Numéro de publication: **0 005 091**
**B1**

(12) # FASCICULE DE BREVET EUROPEEN

(45) Date de publication du fascicule de brevet: **25.11.81**

(21) Numéro de dépôt: **79400193.3**

(22) Date de dépôt: **27.03.79**

(51) Int. Cl.[3]: **C 07 D 407/04,**
C 07 D 277/42,
A 61 K 31/495,
A 61 K 31/425
//C07D295/00

(54) **Nouvelles pipérazines monosubstituées, leurs procédés de préparation et les compositions pharmaceutiques les renfermant.**

(30) Priorité: **13.04.78 GB 1456578**

(43) Date de publication de la demande:
**31.10.79 Bulletin 79/22**

(45) Mention de la délivrance du brevet:
**25.11.81 Bulletin 81/47**

(84) Etats Contractants Désignés:
**BE CH DE FR GB IT NL**

(56) Documents cités:
**FR - A - 2 393 802**
**FR - M - 5 175**

(73) Titulaire: **SCIENCE UNION ET Cie SOCIETE FRANCAISE DE RECHERCHE MEDICALE**
**14 rue du Val d'Or**
**F-92150 Suresnes (FR)**

(72) Inventeur: **Buré, Jacques**
**22, rue Perronet**
**F-92200 Neuilly sur Seine (FR)**
Inventeur: **Regnier, Gilbert**
**Avenue du Plessis**
**F-92290 Chatenay-Malabry (FR)**

(74) Mandataire: **Reverbori, Marcelle et al.**
**SCIENCE UNION ET Cie SOCIETE FRANCAISE DE RECHERCHE MEDICALE Service Brevets 22 rue Garnier**
**F-92200 Neuilly/Seine (FR)**

**EP 0 005 091 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouvelles pipérazines monosubstituées, leurs procédés de préparation et les compositions pharmaceutiques les renfermant.

La présente invention a pour objet des pipérazines monosubstituées, leurs procédés de préparation et les compositions pharmaceutiques les renfermant.

Le brevet special de médicament n° 5175 M décrit à titre de matières premières utilisables pour la synthèse de médicaments, les (thiazolyl-2)-1 pipérazines de formule générale I':

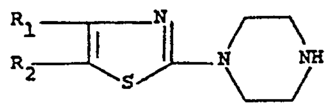

I'

dans laquelle:

$R_1$ représente, entre autres, un radical aralcoyle ayant de 7 à 10 atomes de carbone, et

$R_2$ représente, entre autres, un atome d'hydrogène.

Il a été trouvé, dans les services de la présente demanderesse que ces (thiazolyl-2)-1 pipérazines de formule générale I' et leurs sels d'addition acides physiologiquement tolérables possèdent eux-mêmes des propriétés pharmacologiques et thérapeutiques intéressantes et peuvent de ce fait être utilisés comme médicaments. Cette utilisation nouvelle à titre de médicaments fait partie de la présente invention. Parmi les dérivés intéressants pour cette nouvelle application on peut citer notamment les dérivés de formule générale I' dans laquelle $R_2$ représente un atome d'hydrogène et $R_1$ représente un radical benzyle, o-, m- et p-méthylbenzyle et 1- et 2-phényléthyle. La synthèse de ces dérivés sera illustrée, entre autres, dans les exemples qui suivront.

D'autre part, la présente invention concerne particulièrement les dérivés de formule générale I:

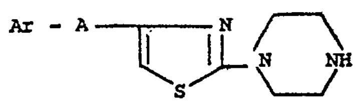

I

dans laquelle:

A représente $-(CH_2)_n-$, dans lequel n est 1, 2, ou 3, ou

—CH—
|
R

dans lequel R représente un radical alkyle ayant de 1 à 5 atomes de carbone, un radical trifluorométhyle ou un radical phényle éventuellement substitué par un ou plusieurs substituants choisis parmi les atomes d'halogène, tels que par exemple les atomes de chlore, brome et fluor, les radicaux alkyle et alkoxy ayant chacun de 1 à 5 atomes de carbone, et le radical trifluorométhyle, et

Ar représente:

un radical phényle substitué par un ou plusieurs substituants choisis parmi les atomes d'halogène tels que par exemple les atomes de chlore, brome et fluor, les radicaux alkoxy ayant de 1 à 5 atomes de carbone et les radicaux méthylènedioxy, hydroxy et trifluorométhyle, ou

un radical de formule générale:

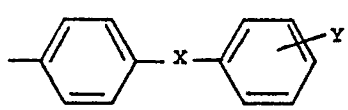

dans laquelle:

X représente une liaison simple, un atome d'oxygène, de soufre ou un radical carbonyle, et

Y représente un atome d'hydrogène, un atome d'halogène, tel que par exemple un atome de chlore, brome ou fluor, ou un radical alkyle ou alkoxy ayant chacun de 1 à 5 atomes de carbone, ou un radical phényle non substitué dans le cas particulier où A représente

—CH—
|
R

dans lequel R prend la signification énoncée précédemment à l'exception du radical alkyle ayant de 1 à 5 atomes de carbone.

La présente invention concerne également les sels d'addition des dérivés de formule générale I avec des acides et plus particulièrement les sels d'addition qui sont physiologiquement tolérables.

La présente invention a également pour objet le procédé de préparation des dérivés de formule générale I caracterisé en ce que l'on condense un dérivé halogéné de formule générale II:

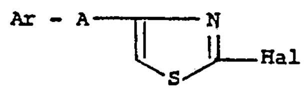

II

dans laquelle Ar et A ont les significations précédemment définies et Hal représente un atome de chlore ou de brome, avec un excès de pipérazine.

Il est avantageux de faire réagir le dérivé de formule II avec la pipérazine en solution dans un alcool aliphatique contenant 4 ou 5 atomes de carbone, à une température comprise entre 110 et 140°C. La quantité de pipérazine utilisée peut être 2 à 5 fois la quantité stoechiométrique, l'excès agissant comme accepteur de l'hydracide formé au cours de la réaction.

La présente invention concerne aussi le procédé de préparation des dérivés de formule générale I caractérisé en ce que l'on condense un dérivé halogéné de formule générale III:

$$Ar—A—CO—CH_2—Hal \qquad III$$

dans laquelle Ar, A et Hal ont les significations précédemment définies,

avec une N-thiocarbamoyl pipérazine N'-substituée de formule générale IV:

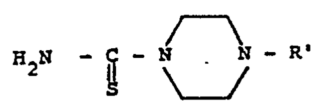

IV

dans laquelle R' est un groupe protecteur tel que par exemple un radical formyle, ou alkoxycarbonyl de préférence éthoxycarbonyle,

puis l'on hydrolyse le composé ainsi obtenu de formule générale V:

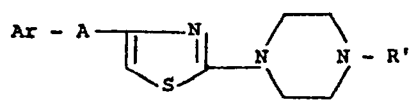

V

dans laquelle Ar, A et R' ont les significations énoncées précédemment.

Il est avantageux d'effectuer la réaction des composés III et IV en solution dans un solvant polaire tel que par exemple un alcool aliphatique ayant 2 à 4 atomes de carbone, de préférence à la température d'ébullition d'un tel mélange, c'est à dire à une température comprise entre 75 et 115°C, puis d'hydrolyser le groupe protecteur avec une base forte, comme par exemple l'hydroxyde de sodium ou de potassium, dans le même solvant.

Les matières premières utilisées pour ces procédés sont des composés connus, ou elles peuvent être preparées selon des méthodes décrites dans la littérature pour préparer des composés analogues, comme mentionné dans les exemples suivants.

Les dérivés de formule générale I sont des bases fortes qui peuvent être transformées par traitement avec les acides en sels d'addition acides. Comme acides qui peuvent être utilisés pour la formation de ces sels, on peut citer par exemple, dans la série minérale, les acides chlorhydrique, bromhydrique, sulfurique et phosphorique et dans la série organique, les acides acétique, propionique, maléique, fumarique, tartrique, citrique, oxalique, benzoïque, méthanesulfonique et iséthionique.

Les dérivés de formule générale I peuvent être purifiés par des méthodes physiques telles que cristallisation ou chromatographie, ou par des méthodes chimiques telles que par exemple la formation des sels d'addition, cristallisation de ces derniers et décomposition par les agents alcalins.

Les dérivés de formule générale I et I' et leurs sels d'addition acides physiologiquements tolérables possèdent des propriétés pharmacologiques et thérapeutiques intéressantes notamment des propriétés antiinflammatoires, principalement lorsque celles-ci sont liées au déclenchement d'une réaction immunitaire secondaire. Ils peuvent, en conséquence, être utilisés comme médicament principalement dans les domaines où il existe des phénomènes inflammatoires dépendant d'une action des lymphocytes, en particulier: en pathologie inflammatoire chronique d'étiologie autoimmune (néphrite, thyroïdite, etc. . .) ou à composante autoimmune comprenant les maladies rhumatoïdes, les collagénoses, la maladie de Orohn, etc. . . , et pathologie infectieuse chronique due aux parasites, bactéries, virus et virus lents avec composants inflammatoire des sphères ORL, respiratoire, urogénitale du système nerveux central ou du tractus digestif (glomérulo néphrite, endocardite, pancréatite avec insulite, hépatite, bronchite chronique etc. . .), en adjuvant des traitements antiinfectieux ou de manière isolée; et dans certaines pathologies inflammatoires et chroniques induites par d'autres hétéro-antigènes (rejet de greffe, dermatite de contact, certains asthmes chroniques, etc. . .)

Leur toxicité est faible et leur $DL_{50}$ déterminée chez la souris par voie orale varie de 250 à plus de 2500 mg/kg selon les composés.

L'activité antiinflammatoire a été mise en évidence, entre autres, par le test de SIEGMUND, E. A. et coll. Proc. Soc. Exp. Biol. Med., (1957), *95*, 729. Lorsque les composés I ou I' sont administrés à la souris par voie orale à la dose de 50 mg/kg on observe des inhibitions des crampes provoquées chez les souris par le phénylbenzoquinone I.P. pouvant atteindre 93%.

L'effet immunomodulateur a été étudié d'après le test d'ASHERSON et coll., Immunology, (1968), *15*, 405. Lorsque les composés I ou I' sont administrés à la souris par voie orale à des doses de 25 à 100 mg/kg, on observe des inhibitions de l'inflammation liée à l'hypersensibilité cutanée induite chez ces souris par l'oxazolone pouvant atteindre 77%.

La présente invention comprend également les compositions pharmaceutiques contenant comme principe actif un dérivé de formule générale I ou I' ou un de leurs sels d'addition acides physiologiquement tolérables mélangé ou associé à un excipient pharmaceutique approprié comme par exemple, l'eau distillée, le glucose, lactose, amidon, talc, éthylcellulose, stéarate de magnésium ou beurre de cacao.

Les compositions pharmaceutiques ainsi obtenues se présentent généralement sous forme dosée et peuvent contenir de 20 à 200 mg de principe actif. Elles peuvent revêtir la forme de comprimés, dragées, gélules, liposomes pour utilisation orale ou injectable, suppositoires, solutions injectables ou buvables, préparations solubles pour infiltration intra-articulaires, pommades, spray, aérosols, et être administrées par voie orale, rectale, parentérale ou locale à la dose de 20 à 200 mg une à quatre fois par jour.

Les exemples suivants illustrent l'invention, les point de fusion étant déterminés dans un tube capillaire sauf-indication contraire.

Exemple 1

(benzyl-4 thiazolyl-2)-1 pipérazine

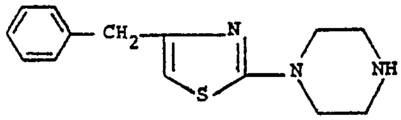

*Première méthode:*

On chaufe pendant 5 heures à ébullition une solution de 14 g de bromo-2 benzyl-4 thiazole ($Eb./_{3mm\ Hg}$: 178—180°C) et de 19 g de pipérazine anhydre dans 200 ml de butanol. Lorsque la réaction est terminée, on filtre le bromhydrate de pipérazine formé et évapore le solvant sous pression réduite. L'excès de pipérazine anhydre est éliminé par sublimation lors du chauffage du résidu sirupeux à 100°C sous une pression réduite de 1 mm de Hg. Le résidu pesant 15 g est traité avec 200 ml d'acétonitrile. On filtre une petite quantité de bromhydrate de pipérazine résiduelle et évapore le solvant sous pression réduite. Le résidu est dissout dans 100 ml d'une solution normale d'acide monométhane sulfonique et la solution obtenue est traitée au charbon actif puis filtrée. Après alcalinisation du filtrat avec un excès de carbonate de potassium, la base libérée est extraite avec deux fois 50 ml de chloroforme et la solution chloroformique obtenue est séchée sur carbonate de potassium. Après évaporation du solvant, on obtient 13 g de cristaux beiges qui recristallisés dans 30 ml de cyclohexane donnent 7,5 g de (benzyl-4 thiazolyl-2)-1 pipérazine sous forme de cristaux blanc crème fondant à 73—74°C.

Le bromo-2 benzyl-4 thiazole de départ a été préparé par réaction de Sandmeyer à partir de l'amino-2 benzyl-4 thiazole, lui-même préparé selon MAHAJANSHETTI et NARGUND, J. Indian Chem. Soc. *39*, 420, (1962) et tous les autres composés de formule II utilisés dans les exemples suivants ont été préparés selon la même méthode.

*Deuxième méthode:*

On chauffe pendant 6 heures à ébullition une solution de 16,9 g de phényl-1 chloro-3 acétone ($Eb/_{0,15mm\ Hg}$: 105—106°C) et de 17,3 g de formyl-1 thiocarbamoyl-4 pipérazine (huile) dans 100 ml d'éthanol. Au bout de ce temps, on ajoute à la solution 6 g de potasse en pastilles et chauffe le mélange 15 heures à l'ébullition pour hydrolyser le groupe protecteur à savoir le radical formyle. On évapore ensuite le solvant sous pression réduite et reprend le résidu par 100 ml d'eau et 150 ml d'une solution normale d'acide monométhane sulfonique. On extrait l'insoluble à l'éther et alcalinise les jus acides par un excès de carbonate de potassium. Après extraction au chloroforme de la base libérée, on évapore le solvant et obtient 7 g de cristaux beiges qui recristallisés dans le cyclohexane, donnent 5 g de (benzyl-4 thiazolyl-2)-1 pipérazine, sous forme de cristaux blanc crème fondant à 73—74°C.

La phényl-1 chloro-3 acétone de départ a été préparée par action du diazométhane sur le chlorure de phénylacétyle dans le tétrahydrofuranne, selon la méthode de MAHAJANSHETTI et NARGUND, J. Indian Chem. Soc. *39*, 420 (1962) et tous les autres composés de formule III utilisés comme matière première dans les exemples suivants ont été préparés selon la même méthode.

La formyl-1 thiocarbamoyl-4 pipérazine de départ a été préparée par analogie avec la méthode décrite par CONROY et DENTON J. Org. Chem. 18, 1489 (1953), à partir du thiocyanate de formyl-1 pipérazine fondant à 118°C.

Exemples 2 à 28

Les dérivés suivants ont été préparés selon la première méthode donnée dans l'exemple 1, à partir d'un excès de pipérazine et de bromo (ou chloro)-2 thiazole substitué en position 4 par un substituant Ar—A— adéquat, et selon la deuxième méthode donnée dans l'exemple 1, à partir de formyl (ou éthoxycarbonyl)-1 thiocarbamoyl-4 pipérazine et de chloro (ou bromo)-3 acétone convenablement substituée en position 1.

2) [(biphénylyl-4 méthyl)-4 thiazolyl-2]-1 pipérazine, P.F. de son dichlorhydrate hemihydrate: 215—220°C, (méhanol/éther).

3) [(chloro-2 benzyl)-4 thiazolyl-2]-1 pipérazine, P.F. de son dichlorhydrate hemihydrate: 175—180°C (éthanol anhydre).

4) [(chloro-3 benzyl)-4 thiazolyl-2]-1 pipérazine, P.F. de son dichlorhydrate hemihydrate: 160—168°C (éthanol-anhydre).

5) [(chloro-4 benzyl)-4 thiazolyl-2]-1 pipérazine, P.F. de son dichlorhydrate hemihydrate: 175—180°C (éthanol anhydre).

6) [(méthyl-2 benzyl)-4 thiazolyl-2]-1 pipérazine.

7) [(méthyl-3 benzyl)-4 thiazolyl-2]-1 pipérazine, P.F. de son hemisulfate hemihydrate: 210—215°C (éthanol anhydre).

8) [(méthyl-4 benzyl)-4 thiazolyl-2]-1 pipérazine, P.F. de son dichlorhydrate hemihydrate: 65—70°C (éthanol anhydre).

9) [(méthoxy-2 benzyl)-4 thiazolyl-2]-1 pipérazine.

10) [(méthoxy-3 benzyl)-4 thiazolyl-2]-1 pipérazine.

11) [(méthoxy-4 benzyl)-4 thiazolyl-2]-1 pipérazine.

12) [(diméthoxy-3,4 benzyl)-4 thiazolyl-2]-1 pipérazine.

13) [(méthylènedioxy-3,4 benzyl)-4 thiazolyl-2]-1 pipérazine.

14) [(trifluorométhyl-3 benzyl)-4 thiazolyl-2]-1 pipérazine.

15) [(phénoxy-4 benzyl)-4 thiazolyl-2]-1 pipérazine, P.F. de son chlorhydrate: 236—242°C (méthanol anhydre/éther).

16) [(p. chlorophénoxy-4 benzyl)-4 thiazolyl-2]-1 pipérazine, P.F. de son dichlorhydrate: 195—200°C (éthanol anhydre).

17) [(phénylthio-4 benzyl)-4 thiazolyl-2]-1 pipérazine, P.F. de son chlorhydrate: 165—170°C (éthanol anhydre).

18) [(benzoyl-4 benzyl)-4 thiazolyl-2]-1 pipérazine, P.F. de son dichlorhydrate hemihydrate: 230—232°C (méthanol à 95%).

19) [($\alpha$-trifluorométhylbenzyl)-4 thiazolyl-2]-1 pipérazine, P.F.: 92—95°C (heptane).

20) [($\alpha$-méthylbenzyl)-4 thiazolyl-2]-1 pipérazine, P.F. de son dichlorhydrate: 212—218°C (éthanol).

21) (benzhydryl-4 thiazolyl-2)-1 pipérazine, P.F. de son dichlorhydrate hydrate: 145—148°C (éthanol anhydre).

22) (phénéthyl-4 thiazolyl-2)-1 pipérazine, P.F. de son dichlorhydrate hemihydrate 220—230°C (éthanol).

23) [(fluoro-4 benzyl)-4 thiazolyl-2]-1 pipérazine, P.F. de son chlorhydrate: 187—192°C (isopropanol anhydre).

24) [(dichloro-2,4 benzyl)-4 thiazolyl-2]-1 pipérazine.

25) [(dichloro-3,4 benzyl)-4 thiazolyl-2]-1 pipérazine.

26) [($\alpha$-méthyl p.chlorobenzyl)-4 thiazolyl-2]-1 pipérazine.

27) [(p.chlorophénylthio-4 benzyl)-4 thiazolyl-2]-1 pipérazine.

28) [(p.chlorobenzoyl-4 benzyl)-4 thiazolyl-2]-1 pipérazine.

Les exemples suivants illustrent les compositions pharmaceutiques renfermant comme principe actif un composé de formule générale I:

Exemple 29

*Gélule dosée à 0,05 g de principe actif:*

| | | |
|---|---|---|
| [(chloro-4 benzyl)-4 thiazolyl-2]-1 pipérazine, dichlorhydrate | 0,05 | g |
| carboxyméthylamidon | 0,005 | g |
| cellulose microcristalline | 0,0808 | g |
| silice colloïdale | 0,0002 | g |

| | | | |
|---|---|---|---|
| | stéarate de magnésium | 0,001 | g |
| | talc | 0,003 | g |

pour une gélule n° 3

Exemple 30

*Comprimé enrobé, dosé à 0,100 g de principe actif:*

| | | | |
|---|---|---|---|
| | [(phénylthio-4 benzyl)-4 thiazolyl-2]-1 pipérazine, chlorhydrate | 0,100 | g |
| | lactose | 0,085 | g |
| | cellulose microcristalline | 0,0502 | g |
| | silice colloïdale | 0,0002 | g |
| | polyvinyl pyrrolidone | 0,010 | g |
| | stéarate de magnésium | 0,0015 | g |
| | talc | 0,005 | g |
| enrobage: | glycérine | 0,00035 | g |
| | hydroxypropylméthylcellulose | 0,00635 | g |
| | laurylsulfate de sodium | 0,00004 | g |
| | oxyde de titane | 0,0019 | g |
| | polyoxyéthylène glycol 6000 | 0,0002 | g |
| | stéarate de magnésium | 0,0002 | g |

Exemple 31

*Gélule dosée à 0,100 g de principe actif:*

| | | | |
|---|---|---|---|
| | [(benzoyl-4 benzyl)-4 thiazolyl-2]-1 pipérazine, dichlorhydrate | 0,100 | g |
| | carboxyméthylamidon | 0,005 | g |
| | cellulose microcristalline | 0,0878 | g |
| | silice colloïdale | 0,0002 | g |
| | stéarate de magésium | 0,001 | g |
| | talc | 0,006 | g |

pour une gélule n° 1

## Revendications

1. Les pipérazines monosubstituées de formule générale:

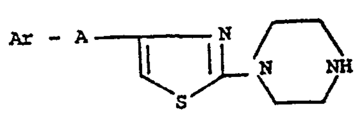

dans laquelle:

A représente $-(CH_2)_n-$ dans lequel n est 1, 2 ou 3, ou

—CH—
|
R

dans lequel R représente un radical alkyle ayant de 1 à 5 atomes de carbone, un radical trifluorométhyle ou un radical phényle éventuellement substitué par un ou plusieurs substituants choisis parmi les atomes d'halogène, les radicaux alkyle et alkoxy ayant chacun de 1 à 5 atomes de carbone et le radical trifluorométhyle, et

Ar représente:

un radical phényle substitué par un ou plusieurs substituants choisis parmi les atomes d'halogène, les radicaux alkoxy ayant de 1 à 5 atomes de carbone et les radicaux méthylènedioxy, hydroxy et trifluorométhyle, ou

un radical de formule:

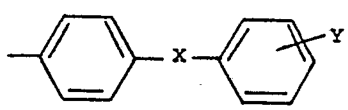

dans laquelle:

X représente une liaison simple, un atome d'oxygène, de soufre ou un radical carbonyle et

Y représente un atome d'hydrogène, un atome d'halogène, ou un radical alkyle ou alkoxy ayant chacun de 1 à 5 atomes de carbone, ou

un radical phényle non substitué dans le cas particulier où A représente

—CH—
|
R

dans lequel R prend la signification énoncée précédemment à l'exception du radical alkyle ayant de 1 à 5 atomes de carbone.

2. Les sels d'addition des composés selon la revendication 1 avec des acides appropriés.

3. Les sels selon la revendication 2 qui sont physiologiquement tolérables.

4. La [(chloro-3 benzyl)-4 thiazolyl-2]-1 pipérazine, et son dichlorhydrate.

5. La [(chloro-4 benzyl)-4 thiazolyl-2]-1 pipérazine, et son dichlorhydrate.

6. La [(phénylthio-4 benzyl)-4 thiazolyl-2]-1 pipérazine, et son chlorhydrate.

7. La [(benzoyl-4 benzyl)-4 thiazolyl-2]-1 pipérazine, et son dichlorhydrate.

8. Un procédé de préparation des composés de la revendication 1, caractérisé en ce que l'on condense un dérivé halogéné de formule générale:

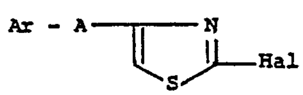 II

dans laquelle Ar et A ont les significations définies dans la revendication 1, et Hal représente un atome de chlore ou de brome, avec un excès de pipérazine.

9. Un procédé de préparation des composés de la revendication 1 caractérisé en ce que l'on condense un dérivé halogéné de formule générale:

$$Ar—A—CO—CH_2—Hal \qquad III$$

dans laquelle Ar et A ont les significations définies dans la revendication 1 et Hal représente un atome de chlore ou de brome, avec une N-thiocarbamoyl pipérazine N'-substituée de formule générale:

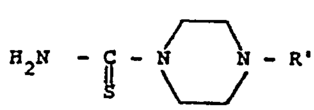 IV

dans laquelle R' est un groupe protecteur tel que par exemple formyle ou alkoxycarbonyle,

puis l'on hydrolyse le composé ainsi obtenu de formule générale V

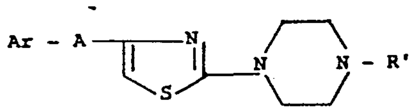 V

dans laquelle Ar, A et R' ont les significations énoncées précédemment.

10. Les compositions pharmaceutiques contenant comme principe actif un composé selon les revendications 1 à 7 avec les supports pharmaceutiques appropriés.

11. Les compositions pharmaceutiques contenant comme principe actif un composé de formule générale I':

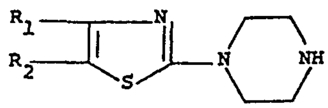

I'

dans laquelle:

$R_1$ représente un radical aralcoyle ayant de 7 à 10 atomes de carbone, et

$R_2$ représente un atome d'hydrogène,

avec des supports pharmaceutiques appropriés.

12. Les compositions pharmaceutiques selon la revendication 11, dans lesquelles le principe actif est:

la (benzyl-4 thiazolyl-2)-1 pipérazine, ou
la [(méthyl-2 benzyl)-4 thiazolyl-2]-1 pipérazine, ou
la [(méthyl-3 benzyl)-4 thiazolyl-2]-1 pipérazine, ou
la [méthyl-4 benzyl)-4 thiazolyl-2]-1 pipérazine, ou
l'[(α-méthylbenzyl)-4 thiazolyl-2]-1 pipérazine, ou
la (phénéthyl-4 thiazolyl-2)-1 pipérazine, ou
un de leurs sels d'addition acides physiologiquement tolérables.

13. Les compositions pharmaceutiques contenant comme principe actif les sels d'addition des composés selon les revendications 11 et 12 avec des acides physiologiquement tolérables, et les supports pharmaceutiques appropriés.

14. Les compositions pharmaceutiques selon les revendications 10 à 13, présentées sous une forme convenant pour l'administration orale, rectale, parentérale ou locale, notamment dans le traitement de l'inflammation à composante immunologique.

**Claims**

1. The monosubstituted piperazines of the general formula:

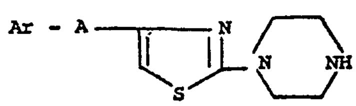

I.

in which:

A represents $—(CH_2)_n—$, in which $n$ is 1, 2, or 3, or

—CH—,
|
R

in which R represents an alkyl radical having from 1 to 5 carbon atoms, a trifluoromethyl radical or a phenyl radical optionally substituted by one or more substituents chosen from halogen atoms, alkyl and alkoxy radicals each having from 1 to 5 carbon atoms and the trifluoromethyl radical, and

Ar represents a phenyl radical substituted by one or more substituents chosen from halogen atoms, alkoxy radicals having from 1 to 5 carbon atoms and the methylenedioxy, hydroxy and trifluoromethyl radicals, or represents a radical of the formula:

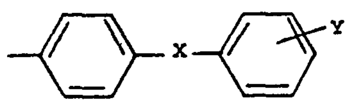

in which:

X represents a single bond, an oxygen atom, a sulphur atom or a carbonyl radical, and

Y represents a hydrogen atom, a halogen atom or an alkyl or alkoxy radical each having from 1 to 5 carbon atoms, or,

in the particular case where A represents

—CH—
|
R

in which R has the meaning given above except for the alkyl radical having from 1 to 5 carbon atoms, an unsubstituted phenyl radical.

2. The addition salts of the compounds according to claim 1 with appropriate acids.

3. The salts according to claim 2 that are physiologically tolerable.

4. 1-[4-(3-chlorobenzyl)-thiazol-2-yl]-piperazine and its dihydrochloride.

5. 1-[4-(4-chlorobenzyl)-thiazol-2-yl]-piperazine and its dihydrochloride.

6. 1-[4-(4-phenylthiobenzyl)-thiazol-2-yl]-piperazine and its hydrochloride.

7. 1-[4-(4-benzoylbenzyl)-thiazol-2-yl]-piperazine and its dihydrochloride.

8. A process for the preparation of the compounds of claim 1, characterised in that a halogen derivative of the general formula:

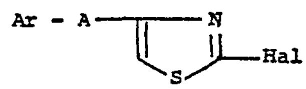

II

in which Ar and A have the meanings defined in claim 1 and Hal represents a chlorine or bromine atom, is condensed with an excess of piperazine.

9. A process for the preparation of the compounds of claim 1, characterised in that a halogen derivative of the general formula:

$$Ar—A—CO—CH_2—Hal \qquad III$$

in which Ar and A have the meanings defined in claim 1 and Hal represents a chlorine or bromine atom, is condensed with an N′-substituted N-thiocarbamoylpiperazine of the general formula:

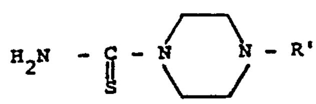

IV

in which R′ is a protecting group, such as, for example, formyl or alkoxycarbonyl, and then the resulting compound of the general formula V

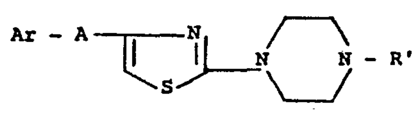

V

in which Ar, A and R′ have the meanings given above, is hydrolysed.

10. The pharmaceutical compositions containing as the active principle a compound according to claims 1 or 2 and 3 to 7 with the appropriate pharmaceutical carriers.

11. Pharmaceutical compositions containing as the active principle a compound of the general formula I′:

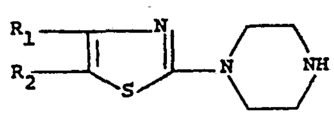

I′

in which

$R_1$ represents an aralkyl radical having from 7 to 10 carbon atoms, and

$R_2$ represents a hydrogen atom,

or one of its salts of addition with physiologically tolerable acids, with appropriate pharmaceutical carriers.

12. The pharmaceutical compositions according to claim 11 in which the active principle is:

1-(4-benzylthiazol-2-yl)-piperazine or

1-[4-(2-methylbenzyl)-thiazol-2-yl]-piperazine or

1-[4-(3-methylbenzyl)-thiazol-2-yl]-piperazine or

1-[4-(4-methylbenzyl)-thiazol-2-yl]-piperazine or

1-[4-($\alpha$-methylbenzyl)-thiazol-2-yl]-piperazine or

1-(4-phenethylthiazol-2-yl)-piperazine.

13. The pharmaceutical compositions containing as the active principle one of the addition salts of the compounds mentioned as active principle in claims 11 and 12, with physiologically tolerable acids.

14. The pharmaceutical compositions according to claims 10 to 13 in a form suitable for oral, rectal, parenteral or local administration, especially in the treatment of inflammation using an immunological component.

**Patentansprüche**

1. Monosubstituierte Piperazine der allgemeinen Formel I

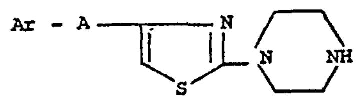

in der

A eine Gruppe der Formel $—(CH_2)_n—$, worin n den Wert 1, 2 oder 3 besitzt, oder der Formel

—CH—,
|
R

worin R für eine Alkylgruppe mit 1 bis 5 Kohlenstoffatomen, eine Trifluormethylgruppe oder eine Phenylgruppe, die gegebenenfalls durch einen oder mehrere Substituenten aus der Halogenatome, Alkyl- und Alkoxy-Gruppen mit jeweils 1 bis 5 Kohlenstoffatomen und Trifluormethylgruppen umfassenden Gruppe substituiert ist, steht; und

Ar eine Phenylgruppe, die durch einen oder mehrere Substituenten aus der Halogenatome, Alkoxygruppen mit 1 bis 5 Kohlenstoffatomen, mit Methylendioxygruppen, Hydroxygruppen und Trifluormethylgruppen umfassenden Gruppe substituiert ist, oder eine Gruppe der Formel

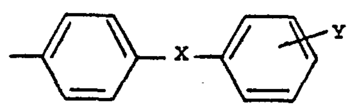

in der

X für eine Einfachbindung, ein Sauerstoffatom, ein Schwefelatom oder eine Carbonylgruppe und

Y für ein Wasserstoffatom, ein Halogenatom oder eine Alkyl- oder Alkoxy-Gruppe mit jeweils 1 bis 5 Kohlenstoffatomen stehen, oder

eine unsubstituierte Phenylgruppe in dem Fall, da A eine Gruppe der Formel

—CH—
|
R

darstellt, worin R die oben angegebenen Bedeutungen mit Ausnahme der Alkylgruppe mit 1 bis 5 Kohlenstoffatomen besitzt, bedeuten.

2. Die Additionssalze der Verbindungen nach Anspruch 1 mit geeigneten Säuren.

3. Die Salze nach Anspruch 1, die physiologisch verträglich sind.

4. 1-[4-(3-Chlorbenzyl)-2-thiazolyl]-piperazin und dessen Dihydrochlorid.

5. 1-[4-(4-Chlorbenzyl)-2-thiazolyl]-piperazin und dessen Dihydrochlorid.

6. 1-[4-(4-Phenylthiobenzyl)-2-thiazolyl]-piperazin und dessen Hydrochlorid.

7. 1-[4-(4-Benzoylbenzyl)-2-thiazolyl]-piperazin und dessen Dihydrochlorid.

8. Verfahren zur Herstellung der Verbindung nach Anspruch 1, dadurch gekennzeichnet, daß man ein Halogenderivat der allgemeinen Formel II

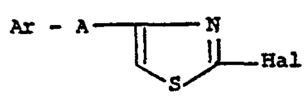

in der Ar und A die in Anspruch 1 angegebenen Bedeutungen besitzen und Hal für ein Chloratom oder ein Bromatom steht, mit einem überschuß Piperazin kondensiert.

9. Verfahren zur Herstellung der Verbindungen nach Anspruch 1, dadurch gekennzeichnet, daß man ein Halogenderivat der allgemeinen Formel III

$$Ar—A—CO—CH_2—Hal$$

in der Ar und A die in Anspruch 1 angegebenen Bedeutungen besitzen und Hal für ein Chloratom oder ein Bromatom steht, mit einem N'-substituierten N-Thiocarbamoyl-piperazin der allgemeinen Formel IV

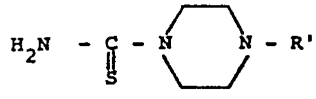

worin R' für eine Schutzgruppe, wie beispielsweise eine Formylgruppe oder eine Alkoxycarbonylgruppe steht, kondensiert und dann die in dieser Weise erhaltene Verbindung der allgemeinen Formel V

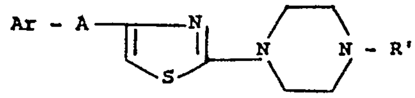

in der Ar, A und R' die oben angegebenen Bedeutungen besitzen, hydrolisiert.

10. Pharmazeutische Zubereitungen enthaltend als Wirkstoff eine Verbindung nach den Ansprüchen 1 oder 2 und 3 bis 7 neben geeigneten pharmazeutischen Trägern.

11. Pharmazeutische Zubereitungen enthaltend als Wirkstoff eine Verbindung der allgemeinen Formel I'

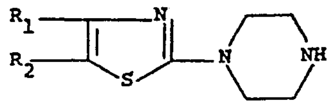

in der

$R_1$ eine Aralkylgruppe mit 7 bis 10 Kohlenstoffatomen und

$R_2$ ein Wasserstoffatom bedeuten,

oder eines ihrer Additionssalze mit physiologisch verträglichen Säuren zusammen mit geeigneten pharmazeutischen Trägern.

12. Pharmazeutische Zubereitungen nach Anspruch 11, dadurch gekennzeichnet, daß sie als Wirkstoff:

1-(4-Benzyl-2-thiazolyl)-piperazin oder
1-[4-(2-Methylbenzyl)-2-thiazolyl]-piperazin oder
1-[4-(3-Methylbenzyl)-2-thiazolyl]-piperazin oder
1-[4-(4-Methylbenzyl)-2-thiazolyl]-piperazin oder
1-[4-($\alpha$-Methylbenzyl)-2-thiazolyl]-piperazin oder
1-(4-Phenyläthyl-2-thiazolyl)-piperazin

enthalten.

13. Pharmazeutische Zubereitungen enthaltend als Wirkstoff ein Additionssalz der als Wirkstoff in den Ansprüchen 11 und 12 genannten Verbindungen mit physiologisch verträglichen Säuren.

14. Pharmazeutische Zubereitungen nach den Ansprüchen 10 bis 13 dadurch gekennzeichnet, daß sie in einer für die Verabreichung auf oralem, rektalem, parenteralem oder lokalem Wege insbesondere zur Behandlung von Entzündungen mit immunologischer Komponente geeigneten Form vorliegen.